# EUROPEAN PATENT SPECIFICATION

(11) **EP 1 700 612 B1**
(45) Date of publication and mention of the grant of the patent: **01.07.2009**
(21) Application number: 06110928.6
(22) Date of filing: 10.03.2006
(51) Int. Cl.: A61M 1/10, A61M 25/10, A61M 1/12

(54) **Aortal pressure controlling device**
Vorrichtung zur Steuerung des Drucks in der Aorta
Dispositif de contrôle de pression aortique

(30) Priority: 11.03.2005 IT BO20050143
(43) Date of publication of application: 13.09.2006
(73) Proprietor: RUNNING HEART LIBERA ASSOCIAZIONE MUTUALISTICA, 31021 MOGLIANO VENETO (IT)
(72) Inventor: Zannoli, Romano, 40138 BOLOGNA (IT)
(74) Representative: Jorio, Paolo

(56) References cited:
- EP-A- 0 402 872
- WO-A-92/11896
- WO-A-03/082379

## Description

The present invention relates to an aortal pressure controlling device.

A disease related to the aorta concerns decreased arterial elasticity. Decreased arterial elasticity necessarily entails a maximum pressure higher than normal and a minimum pressure lower than normal with a consequent reduced coronary perfusion.

The cardiovascular system reacts to this dysfunction by implementing all the possible physiological compensation mechanisms to maintain the average pressure and, therefore, the global blood perfusion within acceptable limits. However, such physiological compensation mechanisms cannot always solve the problem and sometimes have a harmful evolution for the person.

A decreased arterial elasticity therefore plays an important role in the biomechanical behaviour of the cardiovascular system, behaviour which, regretfully, is not easily modified being elasticity an intrinsic feature of the vascular wall.

In particular, aortic dissection is a particularly severe pathology which compromises correct arterial elasticity. Such pathology consists in the laceration, due to traumatic events or degenerative causes, of the innermost part of the arterial wall. Following such laceration, the dissected artery expresses very low pulsability, the ventricle in its ejective phase encounters an aorta with a very low elasticity, and abnormally high systolic pressure levels followed by very low perfusion pressures in the diastolic phase are obtained. The low diastolic pressure negatively effects coronary perfusion and ischemic conditions may occur within the ventricle, being incapable of producing the blood flow rate needed by the organism.

It is the object of the present invention to make a device capable of maintaining, in low arterial elasticity conditions, both the systolic and the diastolic pressure of the aorta within physiological values.

An aortal pressure controlling device according to the preamble of claim 1 is disclosed in EP 0 402 872.

The object of the present invention is an aortal pressure controlling device as defined in claim 1.

With the device according to claim 1 it is possible to correctly adjust the pulsability of the variable volume member within the aorta, to ensure approximation of the maximum and minimum endoaortic pressure to the required average pressure. Furthermore, the device of the present invention is particularly convenient because it adapts automatically to the patient's specific conditions and does not improperly stress the damaged vascular structure.

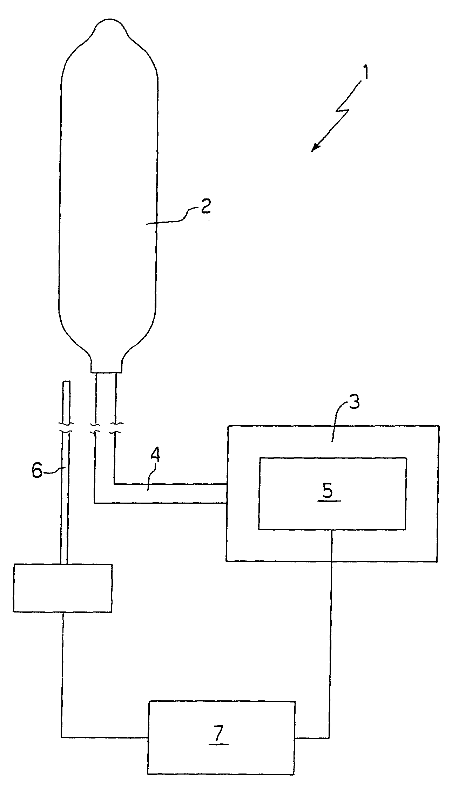

The following is provided by way of illustrative and non-limitative example for better understanding of the invention with the help of the figure in the accompanying drawing, in which the device object of the present invention is partially and schematically shown.

In the figure, it is indicated as a whole by 1 a device according to the present invention in schematic form.

The device 1 comprises a variable volume member 2, a gas reservoir 3 connected to the element 2 by means of a cannula 4 and blowing means 5, which act on the reservoir 3 to adjust blowing of gas inside the member 2.

The device 1 further comprises an aortal pressure measurer 6 and a control unit 7, which is connected both to the measurer 6 and to the blowing means 5.

In use, the member 2, part of the cannula 4 and the measurer 6 are inserted inside the patient's aorta presenting problems of aortal wall reduced elasticity. In the control unit 7 it is entered how much the pressure in the variable volume member 2 must differ, higher or lower, with respect to the patient's average aortal pressure.

Once the measurer 6 detects the aortal pressure, it communicates it to the control unit 7, which compares it with the previously determined datum and consequently operates the blowing means 5 in order to bring the pressure of the variable volume member 2 as close to the predetermined value as possible.

In this manner, the maximum and minimum endoaortic pressure values can be modified, maintaining an average pressure value so as not to cause damage to the patient. Furthermore, for reasons of greater safety, the features of the device 1 ensure prompt reaction of volume variation in the member 2 following external stress, and consequent re-establishment of the required pressure.

The device must be able to allow also the manual operation of the blowing means 5 by an operator, who consequently operates the blowing means 5 after reading the pressure detected by the measurer.

Such possibility ensures a high level of safety, because, in the event of a control unit failure, the required pressure may be however re-established within the aorta.

As may be apparent to a person skilled in the art, the member 2, the blowing means 5 and the pressure measurer may be made in different ways.

Specifically, the blowing means 5 may consist of a piston, whose head is arranged within the reservoir 3 and whose stem comprise a toothed portion to be engaged by a gear wheel or a worm screw, causing the shift of the entire piston.

The gear wheel or worm screw, in turn, is driven by an electrical motor controlled by the control unit according to the data detected from the measurer 6.

The variable volume member 2 may be made in different shapes and of different materials according to needs. Specifically, variable volume members of different dimensions either longitudinal or transversal may be used.

Furthermore, a substance with anti-clotting properties may be applied to the outer wall of the member 2 or the insertion of the element 2 may be associated with the positioning of an aortal stent.

## Claims

1. An aortal pressure controlling device (1) comprising variable volume member (2) with gas inside of it and adapted to being inserted within the aorta, a gas reservoir (3) connected by means of a cannula (4) to said variable volume member (2), blowing means (5) of the gas from said reservoir (3) to said variable volume member (2), an aortal pressure measurer (6), and a control unit (7) connected both to said pressure measurer (6) and to said blowing means (5); said aortal pressure controlling device (1) **being characterized by that** said control unit (7) is configured to operate according to the following steps:
- reception step, in which said control unit (7) receives data from said pressure measurer (6);
- comparison step, in which said control unit (7) compares the data coming from said pressure measurer (6) with data previously entered and relating to how much the pressure in the variable volume member (2) must differ, higher or lower, with respect to the patient's average aortal pressure; and
- pressure adjustment step, in which said control unit (7) operates the blowing means (5) in order to bring the pressure of the variable volume member (2) as close to the data value previously entered as possible.

2. A device according to claim 1, **characterised in that** said blowing means (5) comprise a piston, whose head operates within said reservoir (3).

3. A device according to claim 2, **characterised in that** a stem of said piston is engaged by a gear wheel or a worm screw, moved by an electrical motor controlled by said control unit (7) according to the data detected by the measurer (6).

4. A device according to any of the preceding claims, **characterised in that** said measurer (6) is adapted to be inserted or connected by means of a cannula within the aorta.

5. A device according to any of the preceding claims, **characterised in that** said variable volume element (2) comprises on its outer wall a substance with anti-clotting properties.

## Patentansprüche

1. Regelvorrichtung (1) zur Regelung des Aortadrucks
mit einem Element (2) variablen Volumens, das im Innern Gas enthält und dazu ausgebildet ist, in die Aorta eingeführt zu werden,
mit einem Gasreservoir (3), das mittels einer Kanüle (4) mit dem Element (2) variablen Volumens verbunden ist,
mit einer Aufblasvorrichtung (5) zum Einblasen des Gases aus dem Reservoir (3) in das Element (2) variablen Volumens,
mit einem Druckmessfühler (6) für die Aorta und
mit einer Regeleinheit (7), die sowohl mit dem Druckmessfühler (6) als auch mit der Aufblasvorrichtung (5) verbunden ist,
wobei die Regelvorrichtung (1) zur Regelung des Aortadrucks **dadurch gekennzeichnet ist, dass** die Regeleinheit (7) derart ausgebildet ist, dass sie folgende Schritte ausführt:
- Aufnahmeschritt, in dem die Regeleinheit (7) Daten von dem Druckmessfühler (6) aufnimmt,
- Vergleichsschritt, in dem die Regeleinheit (7) die von dem Druckmessfühler (6) kommenden Daten mit Daten vergleicht, die vorher eingegeben wurden und sich darauf beziehen, wie viel, höher oder niedriger, sich der Druck in dem Element (2) variablen Volumens von dem mittleren Normaldruck der Aorta des Patienten unterscheiden muss, und
- Druckeinstellschritt, in dem die Regeleinheit (7) die Aufblasvorrichtung (5) betätigt, um den Druck des Elements (2) variablen Volumens so nah wie möglich an den Datenwert anzunähern, der vorher eingegeben wurde.

2. Vorrichtung nach Anspruch 1, **dadurch gekennzeichnet, dass** die Aufblasvorrichtung (5) einen Kolben enthält, bei dem die Betätigung des Kopfes innerhalb des Reservoirs (3) vor sich geht.

3. Vorrichtung nach Anspruch 1, **dadurch gekennzeichnet, dass** an einer Stange des Kolbens ein Zahnrad oder eine Antriebsschraube angreift, das bzw. die von einem Elektromotor angetrieben wird, der durch die Regeleinheit entsprechend den Daten gesteuert wird, die durch Druckmessfühler (6) gemessen werden.

4. Vorrichtung nach einem oder mehreren der vorhergehenden Ansprüche, **dadurch gekennzeichnet, dass** der Druckmessfühler (6) dazu ausgebildet ist, in die Aorta eingeführt oder mittels einer Kanüle mit dieser innen verbunden zu werden.

5. Vorrichtung nach einem oder mehreren der vorhergehenden Ansprüche, **dadurch gekennzeichnet, dass** das Element (2) variablen Volumens an seiner Außenwand eine Substanz mit Antigerinnungseigenschaften aufweist.

## Revendications

1. Dispositif de contrôle de pression aortique (1) comprenant un élément à volume variable (2) contenant du gaz et adapté pour être inséré à l'intérieur de l'aorte, un réservoir de gaz (3) relié au moyen d'une canule (4) audit élément à volume variable (2), des moyens de soufflage (5) du gaz depuis ledit réservoir (3) jusqu'audit élément à volume variable (2), un mesureur de pression aortique (6) et une unité de commande (7) connectés tous les deux audit mesureur de pression (6) et audits moyens de soufflage (5) ; ledit dispositif de contrôle de pression aortique (1) étant **caractérisé en ce que** ladite unité de commande (7) est configurée pour fonctionner selon les étapes suivantes :
- étape de réception, dans laquelle ladite unité de commande (7) reçoit des données dudit mesureur de pression (6) ;
- étape de comparaison, dans laquelle ladite unité de commande (7) compare les données venant dudit mesureur de pression (6) aux données entrées précédemment et indiquant de combien la pression dans l'élément à volume variable (2) doit varier, vers le haut ou vers le bas, par rapport à la pression aortique moyenne du patient ; et
- l'étape d'ajustement de pression, dans laquelle ladite unité de commande (7) actionne les moyens de soufflage (5) afin d'amener la pression de l'élément à volume variable (2) aussi près que possible de la valeur de données précédemment entrée.

2. Dispositif selon la revendication 1, **caractérisé en ce que** lesdits moyens de soufflage (5) comprennent un piston, dont la tête fonctionne à l'intérieur dudit réservoir (3).

3. Dispositif selon la revendication 2, **caractérisé en ce qu'**une tige dudit piston est mise en prise par une roue dentée ou une vis sans fin, déplacée par un moteur électrique commandé par ladite unité de commande (7) en fonction des données détectées par le mesureur (6).

4. Dispositif selon l'une quelconque des revendications précédentes, **caractérisé en ce que** ledit mesureur (6) est adapté pour être inséré ou relié au moyen d'une canule à l'intérieur de l'aorte.

5. Dispositif selon l'une quelconque des revendications précédentes, **caractérisé en ce que** ledit élément à volume variable (2) comprend sur sa paroi externe une substance ayant des propriétés anticoagulantes.
